(19) Europäisches Patentamt

European Patent Office

Office européen des brevets

(11) EP 0 848 966 A2

(12) EUROPÄISCHE PATENTANMELDUNG

(43) Veröffentlichungstag:
24.06.1998 Patentblatt 1998/26

(51) Int. Cl.⁶: A61N 5/06

(21) Anmeldenummer: 97122512.3

(22) Anmeldetag: 19.12.1997

(84) Benannte Vertragsstaaten:
AT BE CH DE DK ES FI FR GB GR IE IT LI LU MC NL PT SE
Benannte Erstreckungsstaaten:
AL LT LV MK RO SI

(30) Priorität: 20.12.1996 DE 19653338

(71) Anmelder: Warnke Unit GmbH
66111 Saarbrücken (DE)

(72) Erfinder: Warnke, Ulrich, Dr.
66133 Saarbrücken (DE)

(74) Vertreter:
Bernhardt, Winfrid, Dr.-Ing.
Kobenhüttenweg 43
66123 Saarbrücken (DE)

(54) **Vorrichtung und Verfahren zum Bestrahlen von biologischem Gewebe**

(57)     Die Erfindung betrifft eine Vorrichtung und ein Verfahren zum Bestrahlen von biologischem Gewebe. Gemäß der Erfindung werden wenigstens zwei Lichtstrahlen mit Wellenlängen unterhalb derjenigen des fernen Infrarotbereichs erzeugt. Die Lichtstrahlen werden im Winkel zueinander auf das biologische Gewebe ausgerichtet und in einem gewünschten Bestrahlungsbereich innerhalb des Gewebes überlagert. In dem Bestrahlungsbereich, der aufgrund der Durchdringungsfähigkeit der verwendeten, einander überlagerten Lichtstrahlen unterhalb des Hautgewebes liegen kann, können biochemische Reaktionen, für die Quantenenergien zwischen 3 μm und 25 μm maßgebend sind, stimuliert werden.

FIG. 1

EP 0 848 966 A2

**Beschreibung**

Die Erfindung betrifft eine Vorrichtung und ein Verfahren zum Bestrahlen von biologischem Gewebe, insbesondere zur Beeinflussung von Vorgängen in biologischen Zellen.

Bei den vielfältigen in Zellen ablaufenden biochemischen Reaktionen spielen Absorbtions- und Emissionsvorgänge eine wesentliche Rolle, wobei Moleküle Quanten elektromagnetischer Energie, z.B. unter Eingehen einer Verbindung absorbieren oder emitieren. Dies trifft zum Beispiel für die Verbindung von Enzym und Subtrat, von Antigen und Antikörper sowie von Hormonen und Rezeptoren zu. Beim gegenseitigen Auffinden der diese Aggregate bildenden Molekülbestandteile sind im Zusammenhang mit den Absorptions- und Emissionsvorgängen auftretende Resonanzphänomene von entscheidender Bedeutung. Insbesondere beruht die Wirksamkeit des als Zellenergieträger dienenden AdenosinTriPhosphats (ATP) auf der Speicherung von Energie, die in Form von Quanten elektromagnetischer Energie zugeführt und abgegeben wird.

Die in diesem Zusammenhang maßgebenden Quantenenergien liegen in einem Bereich des elektromagnetischen Spektrums, welcher der Thermostrahlung und angrenzenden Mikrowellenstrahlung mit Wellenlängen zwischen etwa 3 μm und 25 μm entspricht. Diese Strahlung wird bereits in äußeren Schichten des Hautgewebes absorbiert.

Durch die vorliegende Erfindung wird eine Vorrichtung geschaffen, welche eine Einrichtung für die Erzeugung von wenigstens zwei Lichtstrahlen mit Wellenlängen unterhalb des fernen Infrarotbereichs und eine Einrichtung zur Ausrichtung der Lichtstrahlen auf das Gewebe im Winkel zueinander unter Überlagerung in einem gewünschten Bestrahlungsbereich innerhalb des Gewebes aufweist. Die Erfindung schafft ferner ein entsprechendes Verfahren, das durch Erzeugen von wenigstens zwei Lichtstrahlen mit Wellenlängen kleiner als diejenigen des fernen Infrarotbereichs und Richten der Lichtstrahlen auf das Gewebe im Winkel zueinander unter Überlagerung der Lichtstrahlen in einem gewünschten Bestrahlungsbereich innerhalb des Gewebes gekennzeichnet ist.

Der Erfinder hat festgestellt, daß durch Bestrahlung mit sich überlagernden Lichtstrahlen aus dem genannten Wellenlängenbereich Einfluß auf die obengenannten biochemischen Vorgänge in Zellengewebsbereichen weit unterhalb des Hautgewebes genommen werden kann, indem einerseits dieser Wellenlängenbereich Lichtstrahlung mit gegenüber der schon in der Haut absorbierten Ferninfrarotstrahlung höherer Durchdringungsfähigkeit enthält und andererseits die in dem Bestrahlungsbereich aus der Überlagerung der Lichtstrahlen resultierende elektromagnetische Strahlung stimulierend auf die genannten biochemischen Reaktionen einzuwirken vermag.

Vorzugsweise gehören die Lichtstrahlen dem Wellenlängenbereich der nahen Infrarotstrahlung mit Wellenlängen zwischen 750 und 980 nm an, wobei Lichtstrahlen mit Wellenlängen um 900 nm wegen ihrer besonders hohen Eindringfähigkeit in Körpergewebe besonders geeignet sind.

In einer bevorzugten Ausführungsform der Erfindung weist die Lichtstrahlerzeugungseinrichtung zum Bewirken von Interferenzen in dem interessierenden Bestrahlungsbereich wenigstens zwei, kohärentes Licht erzeugende und insbesondere untereinander phasengekoppelte Lichtquellen auf, wobei diese Lichtquellen vorzugsweise Lichtstrahlen mit geringfügig unterschiedlichen Wellenlängen zur Erzeugung von Schwebungsinterferenzen im Bestrahlungsbereich aussenden. Wie der Erfinder herausgefunden hat, kann insbesondere eine solche resultierende elektromagnetische Strahlung mit entsprechend einer Schwebungsfrequenz- bzw. Schwebungswellenlänge an- und abschwellender Amplitude biochemische Reaktionen in den Gewebezellen hervorrufen, wenn die Schwebungswellenlängen in den oben angegebenen Bereich von etwa 3 μm bis 25 μm fallen.

Bei einer Schwebungswellenlänge von 3,24 μm lassen sich insbesondere mit dem AdenosinTriPhosphat (ATP) verbundene Zellreaktionen stimulieren. Das in jeder Zelle gebildete ATP ist die entscheidende Antriebsenergie für alle Lebensprozesse. In 24 Stunden wird insgesamt ca. soviel ATP-Molekülmasse gebildet, wie das eigene Gewicht des Körpers in Kilogramm. Die wichtigste Bedeutung des ATP liegt darin, daß es Enzyme in Zellmembranen, die ATPasen, antreibt, Ionen zu pumpen. So wird das lebensnotwendige Ionenmilieu in der Zelle von z.B. $K^+$, $Na^+$ einerseits und $Ca^{++}$, $Mg^{++}$ andererseits nur dann exakt geregelt, wenn genügende ATP-Energie gebildet werden kann. In diesen Vorgang fließt bis zu 63% der in der Zelle insgesamt gebildeten ATP-Masse. Jede Beeinträchtigung der ATP-Bildung, z.B. durch Kohlenmonoxid im Straßenverkehr, Stress, mangelnde Durchblutung, zuviel Nitratbelastung durch Nahrung und Trinkwasser, falsche Ernährung und vieles mehr, sind die Ionenpumpen deutlich geringer aktiv mit negativen Konsequenzen für die Zellfunktion. Hier kann die eingekoppelte Interferenzstrahlung positiv tätig sein.

Die Lichtstrahlerzeugungseinrichtung weist vorzugsweise Laserlichtquellen auf, insbesondere Laserdioden oder Streulichtdioden mit polarisierendem Filter.

In einer bevorzugten Ausführungsform der Erfindung umfaßt die Lichtstrahlerzeugungseinrichtung wenigstens eine Lichtquelle mit veränderbarer, insbesondere kontinuierlich durchstimmbarer Frequenz auf, und ist ferner mit einer Modulationseinrichtung zur Modulation der Frequenz des Lichts aus dieser Lichtquelle versehen. Durch die Möglichkeit zur Modulation von wenigstens einem der auf das Gewebe gerichteten Lichtstrahlen kann die Schwebungsfrequenz entsprechend moduliert werden, wobei die Wahrscheinlichkeit der Auslösung von Zellenreaktionen dadurch erhöht wird, daß mit der sich ändernden Schwebungsfrequenz eine Resonanzfrequenz eines Zellenmoleküls überstrichen wird.

In weiterer vorteilhafter Ausgestaltung der Erfindung kann vorgesehen sein, daß die Lichtstrahlerzeugungseinrichtung eine Einrichtung für die Erzeugung wenigstens eines gepulsten Lichtstrahls umfaßt, wobei vorzugsweise die Impulsfrequenz und/oder die Impulsdauer variierbar ist. Bevorzugte Impulsfrequenzen liegen im Kilohertz- bis Megahertzbereich. Durch die Pulsung von wenigstens einem der auf das Gewebe gerichteten Lichtstrahlen kann, wie der Erfinder festgestellt hat, eine noch wirksamere Stimulierung erfolgen, indem zwischen den Stimulierungsimpulsen z.B. die weitere Stimulierung begünstigende Regenerationsphasen enthalten sind.

Zur Lichtstrahlpulsung in einem niederfrequenten Bereich kann eine Choppereinrichtung verwendet werden. Möglich ist aber auch die Benutzung von Lichtquellen, die, insbesondere im Kilo- und Megahertzbereich, impulsweise zur Lichtabgabe anregbar sind.

In weiterer vorteilhafter Ausgestaltung der Erfindung ist eine Einrichtung zur Erzeugung eines Magnetfeldes in dem Bestrahlungsbereich vorgesehen. Durch diese Maßnahme wird die Wahrscheinlichkeit der Auslösung von Zellenreaktionen weiter erhöht, indem durch das anliegende Magnetfeld eine Aufspaltung der maßgebenden Energieniveaus in den Molekülen entsprechend dem Zeemann-Effekts erfolgt, so daß z.B. bei variierender Schwebungsfrequenz mehrere Möglichkeiten zur Auslösung einer Resonanzabsorption bestehen.

In weiterer vorteilhafter Ausgestaltung der Erfindung sind die Lichtstrahlen in ihrer Winkelstellung zueinander veränderbar, und auch der Abstand verwendeter Lichtquellen voneinander kann eingestellt werden Durch diese Einstellmöglichkeiten kann einerseits die Tiefenausdehnung des interessierenden Bestrahlungsbereichs geändert werden, andererseits lassen sich die zum Erreichen des Bestrahlungsbereichs erforderlichen Durchstrahlungslängen minimieren.

In weiterer vorteilhafter Ausgestaltung der Erfindung ist eine Einrichtung zur Steuerung des Stromes der Dioden für die Modenfrequenzen des Lichts, der Impulsfrequenz im Kilo- bzw. Megahertzbereich, der Impulsdauer, einer Niederfrequenzmodulation und/oder einer Amplitudenmodulation des Magnetfeldes auf der Grundlage von über einen Datenträger, wie z.B. eine Chipkarte oder Diskette, eingebbaren, anwendungsfallspezifischen Steuerparametern vorgesehen.

In einer weiteren besonderen Ausführungsform der Erfindung wird eine Lichtmeßeinrichtung zur insbesondere frequenzspezifischen Erfassung von Reflektionslichtmengen und einer Einrichtung zur Ermittlung von resultierenden Lichtmengen im Bestrahlungsbereich anhand der erfaßten Reflektionslichtmengen und der Ausgangsparameter der auf das Gewebe gerichteten Lichtstrahlen verwendet. Bei gegebenen Ausgangsparametern lassen sich anhand der ermittelten Reflektionslichtmengen Rückschlüsse daraus ziehen, wie groß die im Bestrahlungsbereich noch ankommenden Lichtmengen sind, so daß z.B. zur Aufrechterhaltung einer bestimmten Intensität innerhalb des Bestrahlungsbereichs die Ausgangsintensität verändert wird.

Weitere vorteilhafte Ausgestaltungsmöglichkeiten der Erfindung gehen aus den Unteransprüchen hervor.

Die Erfindung soll nun anhand von Ausführungsbeispielen und der beiliegenden, sich auf diese Ausführungsbeispiele beziehenden Zeichnungen näher erläutert und beschrieben werden. Es zeigen:

Fig. 1    eine schematische Darstellung eines ersten Ausführungsbeispiels einer erfindungsgemäßen Vorrichtung,

Fig. 2    eine durch die Vorrichtung von Fig. 1 erzeugte Schwebung einer elektromagnetischen Welle, und

Fig. 3    eine weiteres Ausführungsbeispiel für eine erfindungsgemäße Vorrichtung.

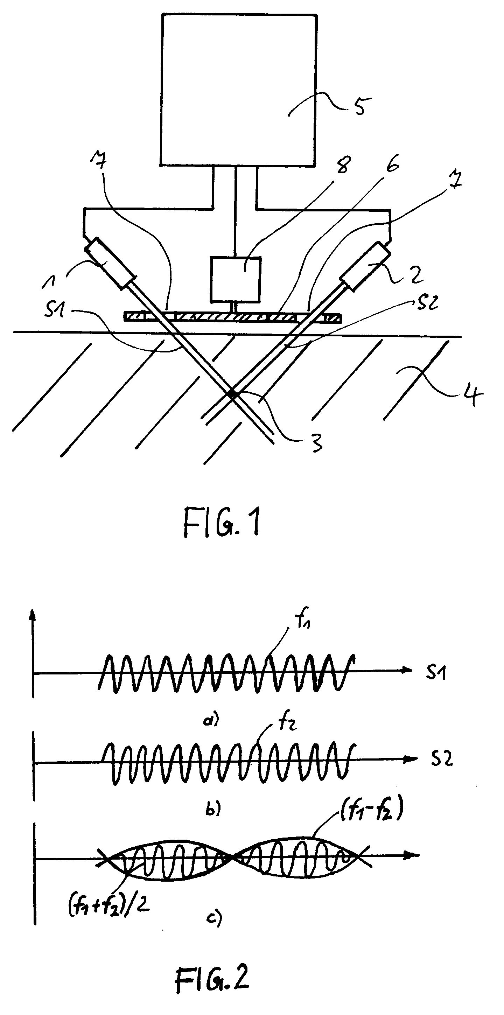

Mit den Bezugszeichen 1 und 2 sind in der Fig. 1 zwei Lichtquellen bezeichnet. Dabei handelt es sich um Laserdioden, die jeweils einen Lichtstrahl S1 und einen Lichtstrahl S2 aussenden. Die Lichtquellen 1 und 2 sind an einer in der Fig. 1 nicht gezeigten Einrichtung gehalten und so ausgerichtet, daß sich die Lichtstrahlen S1 und S2 in einem Bestrahlungsbereich 3 überlagern. Der Bestrahlungsbereich 3 liegt in dem gezeigten Beispiel in einem Körpergewebeabschnitt 4 etwa in einer Tiefe von 5 cm unter der Hautoberfläche.

Die Laserdioden-Lichtquellen 1 und 2 sind jeweils mit einer Versorgungs- und Steuereinheit 5 verbunden sind. Die Laserdioden geben Licht mit einer Wellenlänge um 900 nm ab, wobei sich die Wellenlängen der Lichtquanten im Lichtstrahl S1 geringfügig von den Wellenlängen der Lichtquanten im Lichtstrahl S2 unterscheiden. Die Versorgungs- und Steuereinrichtung 5 ist zum Betrieb der Laserdioden 1 und 2 derart ausgelegt, daß die elektromagnetischen Lichtquellen des Lichtstrahls S1 und des Lichtstrahls S2 in einer festen Phasenbeziehung zueinander stehen oder daß die Phasenbeziehungen in der Zeiteinheit im Zufallsmodus verändert werden.

Mit dem Bezugszeichen 6 ist in der Fig. 1 eine auf einem Kreisumfang im Abstand Öffnungen 7 aufweisende Drehscheibe bezeichnet, die durch einen Motor 8 drehbar ist. Der über eine nicht gezeigte Einrichtung gehalterte Motor 8 ist wie die Laserdioden 1 und 2 mit der Versorgungs- und Steuereinheit 5 verbunden, über die der Motor 8 mit unterschiedlichen Drehzahlen betreibbar ist. Durch die Drehung der Scheibe 6 mit den Öffnungen 7 werden die Lichtstrahlen S1 und S2 aufeinanderfolgend unterbrochen und freigegeben, wobei in dem gezeigten Ausführungsbeispiel die Drehzahl des Motors 8 über die Versorgungs- und Steuereinheit 5 zur Pulsung der Lichtstrahlen S1 und S2 mit Impulsfrequenzen im Kilohertzbereich einstellbar ist. Statt dieser Chopper-Einheit oder zusätzlich kann eine elektronische Ansteuerung die Modulation übernehmen.

Die Versorgungs- und Steuereinheit 5 umfaßt eine Modulationseinrichtung, durch die die Lichtfrequenzen bzw. Lichtwellenlängen der Lichtquanten in wenigstens einem der Lichtstrahlen S1 und S2 unter Beibehaltung eines Frequenzunterschiedes zwischen den Lichtstrahlen kontinuierlich variiert werden können.

Zur Bestrahlung des Gewebeabschnitts 4 werden die Lichtquellen 1 und 2 geeignet ausgerichtet, so daß sich die Lichtstrahlen S1 und S2 an einer gewünschten Stelle in dem Gewebeabschnitt 4 unter Bildung des Bestrahlungsbereiches 3 überschneiden. Zur Ausrichtung der Lichtquellen 1 und 2 sind an der nicht gezeigten Halterungseinrichtung für die Lichtquellen Einstellmöglichkeiten derart vorgesehen, daß sowohl der zwischen den Lichtstrahlen S1 und S2 gebildete Winkel als auch der Abstand der Lichtquellen zueinander verändert werden kann.

Das in dem gezeigten Ausführungsbeispiel für die Lichtstrahlen S1 und S2 verwendete Licht mit Wellenlängen um 900 nm weist für Körpergewebe eine besonders hohe Durchdringungsfähigkeit auf, so daß sich noch nach einer Durchstrahlungslänge von etwa 5 cm für Bestrahlungen ausreichende Lichtintensitäten ergeben. Untersuchungen haben gezeigt, daß bei Bestrahlung von normal durchblutetem Körperabschlußgewebe mit Lichtstrahlung aus dem nahen Infrarotbereich (750 bis 980 nm) bei einer Ausgangsleistung von 25 Watt auf der Achse eines Strahlkegels von 1 cm Durchmesser auf der Haut in der genannten Tiefe von 5 cm eine Restquantenausbeute von $10^4$ Photonen/$cm^2$ auftritt.

Im Bestrahlungsbereich 3 kommt es zu einer Überlagerung der Lichtquanten aus den Lichtstrahlen S1 und S2, wobei die genannte feste Phasenbeziehung und der Frequenzunterschied zwischen den Lichtwellen der beiden Lichtstrahlen so gewählt ist, daß Schwebungen entsprechend Fig. 2 c) auftreten. In diesen Schwebungen schwillt die Amplitude einer durch Überlagerung aus Lichtwellen gemäß Fig. 2 a) und b) gebildeten Lichtwelle mit der Frequenz (f1 + f2)/2 an und ab, wobei f1 die Lichtwellenfrequenz im Lichtstrahl S1 und die f2 die Lichtwellenfrequenz im Lichtstrahl S1 bezeichnet. Das An- und Abschwellen erfolgt entsprechend der Schwebungsfrequenz (f1 - f2).

Wie sich herausgestellt hat, sind insbesondere die in Fig. 2 c) gezeigten Schwebungen zur Stimulierung biochemischer Reaktionen in den Zellen des Körpergewebes geeignet. Wählt man den Frequenzunterschied zwischen den Quanten der Lichtstrahlen S1 und S2 derart, daß sich Schwebungsfrequenzen um 3,24 μm ergeben, so können gezielt mit AdenosinTriPhosphat (ATP) verbundene Zellenreaktionen ausgelöst werden. Die Quantenenergie, um ATP zu bilden, liegt bei 0,39 eV (= 9 kcal/mol). Um notwendige optisch-mechanische Photon-Phonon-Beziehungen (elektromagnetische Wellen plus Schallwellen ergeben im Molekül Solitonen) anzuregen, bedarf es einer Wellenlänge von 3,24 μm, die mit dem Umgebungsmilieu allerdings Verschiebungen erhält. Der Erfolg einer verstärkten ATP-Aktivität besteht z.B. meßbar in einer erhöhten Autorhythmik der arteriellen Blutgefäße um ca. 70%, wodurch Blut an kritischen Stellen leichter transportiert wird; außerdem wird die Zellmembran hyperpolarisiert, wodurch bei Nervenzellen diverse Erregungen abgeblockt werden können, ähnlich einer Anästhesie. Weiterhin werden diverse Enzyme aktiver und dadurch kann Wachstum, z.B. von Pflanzen, und Stoffwechselprozesse forciert werden.

Indem durch die Modulation der Lichtwellenlänge von wenigstens einem der Lichtstrahlen S1 und S2 die Schwebungsfrequenz (f1 - f2) laufend geändert wird kann z.B. eine für die Auslösung einer biochemischen Reaktion maßgebende Resonanzfrequenz überstrichen, somit durch eine solche Wellenlängenmodulation die Wahrscheinlichkeit für die Auslösung derartiger Reaktionen erhöht und letztlich die Bestrahlungseffizienz gesteigert werden.

Auch die Pulsung der Lichtstrahlen S1 und S2 trägt zur Erhöhung der Bestrahlungseffizienz bei. Alle Prozesse der Anregung haben eigene Absorptions- und Anregungsausbreitungszeiten. Eine zu schnelle Pulsung der Anregung ergibt einen Energiestau, da die Energie sich nicht schnell genug verbreiten kann. Zum Beispiel beträgt die thermische Diffusionslänge der Anregung $\mu = \sqrt{2\alpha/\infty}$; die Leitzahl für elektromagnetische Schwingungen im Bereich 10 μm beträgt ca. $\alpha = 1,4 \times 10^{-7}$ $m^2$/sec für biologisches Gewebe. Nach Durchlaufen dieser Wegstrecke ist die Amplitude auf den 1/e ten Teil abgedampft. Die Pulsung muß diese Daten berücksichtigen.

Es zeigt sich auch überraschenderweise, daß eine weitere Pulsung des Lichtes im extremen Niederfrequenzbereich zwischen 3 Hz und 300 Hz die Eindringtiefe der Strahlung erhöht.

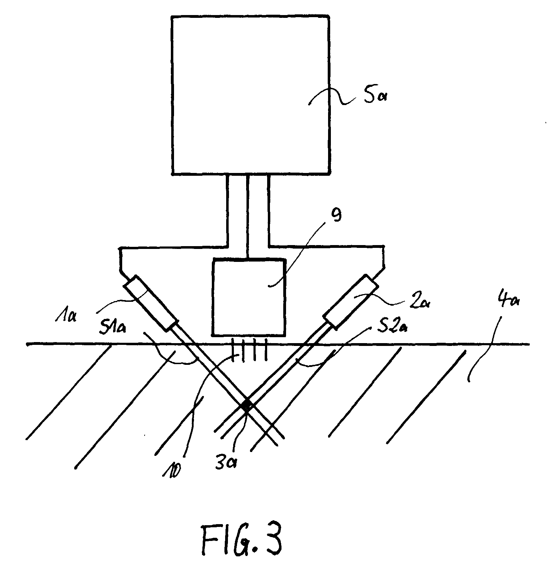

Es wird nun auf Fig. 3 Bezug genommen, wo dem vorangehend beschriebenen Ausführungsbeispiel entsprechende Teile mit der gleichen, jedoch mit dem Buchstaben a versehenen Bezugszahl bezeichnet sind.

Das in der Fig. 3 gezeigte weitere Ausführungsbeispiel unterscheidet sich von dem Ausführungsbeispiel gemäß Fig. 1 dadurch, daß eine Pulsung von Lichtstrahlen S1a und S2a nicht über eine gedrehte Lochscheibe erfolgt, sondern eine Versorgungs- und Steuereinheit 5a so ausgelegt ist, daß Lichtquellen 1a und 2a zur impulsweisen Erzeugung von Lichtstrahlen aktiviert werden. Darüberhinaus ist in der Vorrichtung von Fig. 3 eine Spule 9 zur Erzeugung eines Magnetfeldes 10 vorgesehen, wobei das Magnetfeld 10 einen Körpergewebeabschnitt 4a in einem gewünschten Bestrahlungsbereich 3a, wo sich die Lichtstrahlen S1a und S2a überschneiden, anliegt.

Durch das anliegende Magnetfeld kann es in den in Frage kommenden Molekülen zu einer Aufspaltung von Energieniveaus entsprechend dem Zeemann-Effekt kommen, so daß insbesondere bei der beschriebenen Variation von Schwebungsfrequenzen die Wahrscheinlichkeit zur Auslösung einer Zellenreaktion infolge der Vermehrung der Absorptions- und Emissionsmöglichkeiten durch die Aufspaltung der Energieniveaus erhöht ist.

Die mit der Versorgungs- und Steuereinheit 5a verbundene Magnetspule 9 kann durch die Steuereinheit 5a auch so betrieben werden, daß ein variierendes Magnetfeld erzeugt wird. Durch diese Maßnahme läßt sich die Wahrscheinlichkeit für die Auslösung von biochemischen Reaktionen im Zusammenwirken mit einer Variation der Schwebungsfrequenz weiter steigern.

**Patentansprüche**

1. Vorrichtung zur Bestrahlung von biologischem Gewebe,
   gekennzeichnet durch eine Einrichtung für die Erzeugung von wenigstens zwei Lichtstrahlen (S1,S2) mit Wellenlängen unterhalb derjenigen des fernen Infrarotbereichs und einer Einrichtung zur Ausrichtung der Lichtstrahlen (S1,S2) auf das Gewebe (4) unter Überlagerung in einem gewünschten Bestrahlungsbereich (3).

2. Vorrichtung nach Anspruch 1,
   dadurch gekennzeichnet,
   daß die Lichtstrahlen (S1,S2) Wellenlängen im Bereich von 750 nm bis 980 nm aufweisen.

3. Vorrichtung nach Anspruch 1 oder 2,
   dadurch gekennzeichnet,
   daß die Lichtstrahlen (S1,S2) Wellenlängen um 900 nm aufweisen.

4. Vorrichtung nach einem der Ansprüche 1 bis 3,
   dadurch gekennzeichnet,
   daß die Lichtstrahlerzeugungseinrichtung zur Erzeugung von Interferenzen in dem Bestrahlungsbereich (3) wenigstens zwei, kohärentes Licht und insbesondere untereinander phasengekoppelte Lichtwellen erzeugende Lichtquellen umfaßt.

5. Vorrichtung nach einem der Ansprüche 1 bis 4,
   dadurch gekennzeichnet,
   daß die Lichtstrahlen geringfügig unterschiedliche Frequenzen f1 und f2 derart aufweisen, daß Schwebungsinterferenzen mit Schwebungswellenlängen (c/(f1 - f2)) im Bereich von 3 bis 25 μm erzeugbar sind.

6. Vorrichtung nach Anspruch 5,
   dadurch gekennzeichnet,
   daß Schwebungswellenlängen von etwa 3,24 μm erzeugbar sind.

7. Vorrichtung nach einem der Ansprüche 1 bis 6,
   dadurch gekennzeichnet,
   daß die Lichtstrahlerzeugungseinrichtung Laserlichtquellen, insbesondere Laserdioden (1,2), aufweist.

8. Vorrichtung nach einem der Ansprüche 1 bis 7,
   dadurch gekennzeichnet,
   daß die Lichtstrahlerzeugungseinrichtung wenigstens eine Lichtquelle (1,2) mit veränderbarer, insbesondere kontinuierlich durchstimmbarer Lichtwellenfrequenz aufweist.

9. Vorrichtung nach Anspruch 8,
   dadurch gekennzeichnet,
   daß die Lichtstrahlerzeugungseinrichtung eine Modulationseinrichtung zur Modulation der Lichtwellenfrequenz der wenigstens einen Lichtquelle umfaßt.

10. Vorrichtung nach einem der Ansprüche 1 bis 9,
    dadurch gekennzeichnet,
    daß die Lichtstrahlerzeugungseinrichtung eine Einrichtung (6-8) zur Pulsung wenigstens eines der Lichtstrahlen (S1,S2) umfaßt.

11. Vorrichtung nach Anspruch 10,
    dadurch gekennzeichnet,
    daß die Lichtstrahlpulsungseinrichtung eine Choppereinrichtung umfaßt.

12. Vorrichtung nach Anspruch 10,
    dadurch gekennzeichnet,
    daß die Lichtstrahlpulsungseinrichtung eine Versorgungs- und Steuereinrichtung zur impulsweisen Anregung einer Lichtquelle aufweist.

13. Vorrichtung nach einem der Ansprüche 10 bis 12,
    dadurch gekennzeichnet,
    daß die Impulsfrequenz und/oder Impulsdauer variierbar ist.

14. Vorrichtung nach einem der Ansprüche 10 bis 13,
    dadurch gekennzeichnet,
    daß die Lichtstrahlpulsungseinrichtung zur Erzeugung von Impulsen mit Frequenzen im Kiloherz- und/oder Megaherzbereich und insbesondere für die Erzeugung einer weiteren Pulsung im Bereich von 3 bis 300 Hz vorgesehen ist.

15. Vorrichtung nach einem der Ansprüche 1 bis 14,
    dadurch gekennzeichnet,
    daß eine Einrichtung (9) zur Erzeugung eines Magnetfeldes (10) in dem Bestrahlungsbereich (3a) vorgesehen ist.

16. Vorrichtung nach Anspruch 15,
    dadurch gekennzeichnet,
    daß die Feldstärke des Magnetfelds variierbar ist.

**17.** Vorrichtung nach einem der Ansprüche 1 bis 16, dadurch gekennzeichnet, daß die Ausrichteinrichtung zur Verstellung des Abstandes zwischen die Lichtstrahlen (S1,S2) erzeugenden Lichtquellen und des Winkels zwischen den Lichtstrahlen (S1,S2) vorgesehen ist.

**18.** Vorrichtung nach einem der Ansprüche 1 bis 17, dadurch gekennzeichnet, daß die Intensität der Lichtstrahlen auf der Hautoberfläche im Bereich von 50 bis 1000 mW/cm$^2$ und vorzugsweise bei 500 mW/cm$^2$ liegt.

**19.** Vorrichtung nach einem der Ansprüche 1 bis 18, dadurch gekennzeichnet, daß der Strahldurchmesser der Lichtstrahlen auf der Hautoberfläche etwa bei 3 mm liegt.

**20.** Vorrichtung nach einem der Ansprüche 15 bis 19, dadurch gekennzeichnet, daß eine Einrichtung zur Steuerung des Stromes der Dioden für die Modenfrequenz des Lichts, der Impulsfrequenz im Kilo- bzw. Megahertzbereich, der Impulsdauer, einer Niederfrequenzmodulation und/oder einer Amplitudenmodulation des Magnetfelds auf der Grundlage von über einen Datenträger, wie z.B. eine Chipkarte oder Diskette, eingebbaren, anwendungsfallspezifischen Steuerparametern vorgesehen ist.

**21.** Vorrichtung nach einem der Ansprüche 1 bis 20, dadurch gekennzeichnet, daß eine Lichtmeßeinrichtung zur insbesondere frequenzspezifischen Erfassung von Reflektionslichtmengen und eine Einrichtung zur Ermittlung von resultierenden Lichtmengen im Bestrahlungsbereich anhand der erfaßten Reflektionslichtmengen und der Ausgangsparameter der auf das Gewebe gerichteten Lichtstrahlen vorgesehen ist.

**22.** Verfahren zur Bestrahlung von biologischem Gewebe, gekennzeichnet durch Erzeugen von wenigstens zwei Lichtstrahlen (S1,S2) mit Wellenlängen unterhalb derjenigen des fernen Infrarotbereichs und Richten der Lichtstrahlen auf das Gewebe (4) im Winkel zueinander unter Überlagerung der Lichtstrahlen in einem gewünschten Bestrahlungsbereich (3) innerhalb des Gewebes (4).

**23.** Verfahren nach Anspruch 22, dadurch gekennzeichnet, daß zur verstärkten Erzeugung von Interferenzen Lichtstrahlen (S1,S2) mit kohärenten und insbesondere von Lichtstrahl zur Lichtstrahl phasengekoppelten Lichtwellen erzeugt werden.

**24.** Verfahren nach Anspruch 23, dadurch gekennzeichnet, daß Lichtstrahlen (S1,S2) mit geringfügig unterschiedlichen Frequenzen für die Erzeugung von Schwebungsfrequenzen erzeugt werden.

**25.** Verfahren nach Anspruch 24, dadurch gekennzeichnet, daß Schwebungsinterferenzen mit Schwebungswellenlängen zwischen etwa 3 und 25 μm und insbesondere von etwa 3,24 μm erzeugt werden.

**26.** Verfahren nach einem der Ansprüche 22 bis 25, dadurch gekennzeichnet, daß Lichtstrahlen (S1,S2) mit Wellenlängen im Bereich von 750 bis 980 nm und insbesondere um 900 nm erzeugt werden.

FIG. 1

FIG.2

FIG. 3